(19) Europäisches Patentamt
European Patent Office
Office européen des brevets

(11) **EP 2 412 431 B1**

(12) **EUROPEAN PATENT SPECIFICATION**

(45) Date of publication and mention
of the grant of the patent:
**07.02.2018 Bulletin 2018/06**

(21) Application number: **10756017.9**

(22) Date of filing: **19.03.2010**

(51) Int Cl.:
***A61K 9/107*** *(2006.01)*

(86) International application number:
**PCT/JP2010/054855**

(87) International publication number:
**WO 2010/110220 (30.09.2010 Gazette 2010/39)**

(54) **DISPERSION AND MANUFACTURING METHOD THEREFOR**

DISPERSION UND HERSTELLUNGSVERFAHREN DAFÜR

DISPERSION ET SON PROCÉDÉ DE FABRICATION

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **23.03.2009 JP 2009070829**

(43) Date of publication of application:
**01.02.2012 Bulletin 2012/05**

(73) Proprietor: **FUJIFILM Corporation
Tokyo 106-8620 (JP)**

(72) Inventor: **MORI, Hisahiro
Ashigarakami-gun
Kanagawa 258-8577 (JP)**

(74) Representative: **Hoffmann Eitle
Patent- und Rechtsanwälte PartmbB
Arabellastraße 30
81925 München (DE)**

(56) References cited:
**WO-A1-2007/060174          WO-A1-2007/060177
JP-A- 2007 270 073          JP-A- 2008 154 577
JP-A- 2008 280 257          JP-A- 2009 201 371**

**Description**

Technical Field

The present invention relates to a dispersion composition and a method for manufacturing a dispersion composition.

Background Art

Recently, focusing on the functionality of polyphenol compounds such as catechins or vegetable pigments, many compositions containing such polyphenol compounds have been developed. Examples of compositions containing a polyphenol compound include those obtained by mixing components containing a polyphenol compound in advance, as well as those obtained by preparing components other than a polyphenol compound and then adding a polyphenol compound thereto.

For example, in Japanese Patent Application Laid-Open (JP-A) No. 2000-136123, an external preparation for skin containing a metal chelating agent, an ultraviolet protecting agent, an active oxygen eliminator and the like is disclosed, and examples of the active oxygen eliminator include catechins.

In JP-A No. 2001-316259, a polyphenol formulation is disclosed which is obtained by reducing in size in an oil containing a polyhydric alcohol fatty acid ester, and then emulsifying this oil into an oil-in-water type in the presence of the polyhydric alcohol fatty acid ester. It is described that the polyphenol formulation has a microcapsule structure in which a homogeneous oil coating agent layer is formed on the surface of microparticles of the polyphenol, and that the polyphenol formulation exhibits excellent water dispersibility.

When a fat-soluble substance is used in an emulsified state in combination with a polyphenol compound, the emulsified substance and the polyphenol compound tend to aggregate, which is problematic. In order to overcome this problem, the emulsion stability of the fat-soluble substance has been subject to further improvement.

For example, JP-A No. 2008-280257 discloses, as an emulsion composition having excellent emulsion stability, an emulsion composition which contains a fat-soluble substance, a phospholipid and a sucrose fatty acid ester, and in which the mass ratio of a polyglycerin fatty acid ester to the sucrose fatty acid ester is 0.1 or lower. According to this document, the emulsion composition does not undergo aggregation or the like even when the composition is brought into contact with a polyphenol compound.

On the other hand, JP-A No. 2005-320264 discloses a gel base for external use containing a polyphenol in a water-soluble polymer. This gel base for external use contains a polyphenol in a water-soluble polymer in order to heighten the gel strength of the gel composition.

However, among polyphenols, poorly water-soluble polyphenol compounds are known to be very difficult to emulsify or disperse and are also known to lack stability after dispersion. Therefore, it has been extremely difficult to stably disperse a poorly water-soluble polyphenol compound by using the same formulation as for a soluble polyphenol compound.

WO 2007/060177 A1 and WO 2007/060174 A1 disclose an oil-in-water emulsion in which the dispersed oil droplets exhibit a self-assembled structure.

JP 2008-154577 A discloses an emulsified composition comprising emulsified particles having small particle diameter.

JP 2007-270073 A discloses an emulsion composition containing a pigment originating from hematococcus.

JP 2009-201371 A discloses a curcumin composition comprising dispersing particles containing curcumin, an emulsifier, and an aqueous solvent.

SUMMARY OF INVENTION

Technical Problem

An object of the present invention is to provide a dispersion composition including a poorly water-soluble polyphenol compound and having excellent dispersion stability. Solution to Problem

The present invention provides a dispersion composition and a method for manufacturing the same.

The first aspect of the present invention provides a dispersion composition which includes a poorly water-soluble polyphenol compound, an emulsifier including a sucrose fatty acid ester, and a water-soluble polymer, and, in the dispersion composition, a content of a polyglycerin fatty acid ester in the composition is 0, or 0.1 or fewer times a total mass of the sucrose fatty acid ester in the composition, and a particle size of dispersed particles including the poorly water-soluble polyphenol is 200 nm or smaller.

The poorly water-soluble polyphenol compound is at least one selected from the group consisting of resveratrol, curcumin, rutin, ellagic acid and quercetin.

The water-soluble polymer is at least one selected from the group consisting of proteins and polysaccharides.

The second aspect of the present invention provides cosmetic compositions, food compositions or pharmaceutical compositions including the dispersion composition.

The third aspect of the present invention provides a method for manufacturing the dispersion composition, the method including: an oil phase preparation process in which an oil phase is prepared by dissolving one or more oil phase components including a poorly water-soluble polyphenol in a good solvent for the poorly water-soluble polyphenol compound; and a mixing process in which the obtained oil phase and a phase of a poor solvent for the poorly water-soluble polyphenol compound are mixed to obtain the dispersion composition which includes the poorly water-soluble polyphenol compound and includes dispersed particles having an average volume particle size of 200 nm or smaller.

The good solvent for the poorly water-soluble polyphenol compound may be a water-soluble organic solvent or an aqueous alkali solution.

The mixing of the oil phase and the phase of the poor solvent may be performed by allowing the oil phase and the phase of the poor solvent to pass through a microchannel of from 1 $\mu m^2$ to 1 $mm^2$ individually, and then combining and mixing the oil phase and the phase of the poor solvent.

BRIEF DESCRIPTION OF DRAWINGS

[Fig. 1] Fig. 1 is an exploded perspective view of a microdevice as one example of a micromixer.
[Fig. 2] Fig. 2 is a schematic cross sectional view of a T-shaped microreactor showing one example of a mixing mechanism by a T-shaped microreactor.
[Fig. 3] Fig. 3 is a conceptual diagram of a T-shaped microreactor showing one example of a mixing mechanism by a T-shaped microreactor.

DESCRIPTION OF EMBODIMENT

The dispersion composition of the present invention is a dispersion composition which includes a poorly water-soluble polyphenol compound which is at least one selected from the group consisting of resveratrol, curcumin, rutin, ellagic acid and quercetin, an emulsifier including a sucrose fatty acid ester, and a water-soluble polymer which is at least one selected from the group consisting of proteins and polysaccharides, and the content of a polyglycerin fatty acid ester in the composition is 0, or 0.1 or fewer times the total mass of the sucrose fatty acid ester in the composition, and the particle size of dispersed particles including the poorly water-soluble polyphenol is 200 nm or smaller.

In the dispersion composition of the present invention, by including an emulsifier which includes a sucrose fatty acid ester and in which a compounding ratio of polyglycerin fatty acid esters to sucrose fatty acid esters is extremely low, and a water-soluble polymer, the poorly water-soluble polyphenol compound can be stably dispersed as fine dispersed particles.

The dispersion composition of the present invention is configured to have a form of an O/W emulsified product in which dispersed particles including a poorly water-soluble polyphenol compound are dispersed as an oil phase in a water phase. Here, the poorly water-soluble polyphenol compound may constitute a part of the dispersed particles. The average particle size of the dispersed particles of the present invention means the average particle size of the total oil droplet-like dispersed particles dispersed in the water phase.

The term "process" herein means not only an individual process but also a process in which an expected effect in the process is attained even when the process cannot be clearly distinguished from other processes.

The range of numerical value represented by "from A to B" means a range including A and B as the minimum value and the maximum value respectively.

In the present invention, when the amount of each of the components of the composition is referred to, in case where there are plural substances corresponding to each of the components of the composition, unless otherwise specified, the amount of each of the components of the composition means the total amount of the plural substances present in the composition.

The present invention is described in the following.

[Poorly water-soluble polyphenol compound]

The poorly water-soluble polyphenol compound of the present invention means a polyphenol compound having a solubility in pure water at a temperature of 25°C of 0.1% by mass or lower. Examples of the poorly water-soluble polyphenol compound include a polyphenol compound having, in the same conditions, a solubility in water of from 0.001% by mass to 0.1% by mass.

The polyphenol compound is a compound derived from plants and having two or more phenolic hydroxyl groups in a single molecule. Examples of the polyphenol compound include flavonoids (catechin, anthocyanins, flavone, isoflavone, flavan, flavanone, rutin), phenolic acids (chlorogenic acid, ellagic acid, gallic acid, propyl gallate), lignans, curcumins and coumarins. Among these, those having the above solubility to water fall into the poorly soluble polyphenol compound of the present invention.

Examples of such a poorly water-soluble polyphenol compound include resveratrol, curcumin, rutin, ellagic acid, quercetin and combinations thereof. Among others, those having a solubility also in the below-described water-soluble organic solvent are further preferred. Having a solubility in a water-soluble organic solvent in this case means having a solubility in a water-soluble organic solvent at 25°C of 0.1% by mass or higher, preferably 1.0% by mass or higher. From the viewpoint of a solubility in a water-soluble organic solvent, examples of a preferred poorly water-soluble polyphenol compound include resveratrol, curcumin, rutin, quercetin or a combination thereof.

The poorly water-soluble polyphenol compound may be contained in the dispersion composition of the present invention as a compound. The poorly water-soluble polyphenol compound may be used in a state of a naturally-derived extract as described below.

Examples of naturally-derived extracts include a licorice extract, a cucumber extract, a Millettia reticulata extract, a gentian extract, a Geranium thunbergii extract, a cholesterol and its derivatives, a hawthorn extract, a peony extract, a gingko extract, a Baikai skullcup (Scutellariae Radix extract), a carrot extract, a rugosa rose (Maikai) extract, a Chamaecrista nomame extract, a Tormentil extract, a parsley extract, a peony extract, a Japanese quince extract, a Melissa extract, an alnus firma fruit extract, a strawberry geranium extract, a rosemary extract, a lettuce extract, a tea extract (oolong tea, black tea, green tea and the like), a microbial fermentation metabolic product and a Momordica grosvenori Swingle extract (in parentheses, a synonym of the plant, a herbal medicine name, or the like is described).

The content of the poorly water-soluble polyphenol compounds in the dispersion composition of the present invention is not particularly limited, and is preferably, from the viewpoint of exerting functionality as a polyphenol compound, from 15% by mass to 50% by mass, and more preferably from 20% by mass to 30% by mass.

[Water soluble polymer]

As a water soluble polymer in the dispersion composition of the present invention, a protein or a polysaccharide which can dissolve in water (25°C) by at least about 0.001% by mass is used. In the present invention, by the presence of a water-soluble polymer in the dispersion composition together with the below-described specific emulsifier including a sucrose fatty acid ester, the dispersibility with respect to a poorly water-soluble polyphenol compound can be stabilized. The molecular weight in terms of a weight-average molecular weight of the water-soluble polymer is preferably from 1,000 to 600,000, and more preferably, from the viewpoint of dispersion stability, from 1,000 to 100,000.

Examples of the water soluble polymer which can be used in the present invention include polysaccharides such as pectins, kappa carrageenan, locust bean gum, guar gum, hydroxypropyl guar gum, xanthan gum, karaya gum, tamarind seed polysaccharide, gum arabic, tragacanth gum, hyaluronic acid, sodium hyaluronate, sodium chondroitin sulfate and sodium alginate; proteins such as casein, albumin, methylated collagen, hydrolyzed collagen, water-soluble collagen and gelatin; water soluble cellulose derivatives such as hydroxyethyl cellulose and methyl cellulose; and combinations of two or more thereof. These may be those synthesized or natural products. Among others, from the viewpoint of the fineness of the dispersed particles and the temporal stability of the dispersion composition, at least one selected from the group consisting of polysaccharides and proteins is used.

Among the above proteins, collagen derivatives such as gelatin are preferred. From the viewpoint of the stability of the dispersed particles and the suitability for the process, as the collagen derivatives, those having a weight-average molecular weight of 200,000 or less are preferred, and those having a weight-average molecular weight in the range of from 1,000 to 100,000 are more preferred.

Preferable specific examples of the polysaccharides include kappa carrageenan, dextran and hyaluronic acids.

As the kappa carrageenan and the dextran, those having a weight-average molecular weight of 600,000 or less are preferred, and the molecular weight is more preferably in the range of from 10,000 to 300,000.

As the hyaluronic acids, those having a weight-average molecular weight of 300,000 or less are preferably used. A more preferred molecular weight of the hyaluronic acids is in the range of from 5,000 to 200,000. As the weight-average molecular weight of these polymers, values determined by a gel permeation chromatography are used.

As the water-soluble polymer of the present invention, from the viewpoint of the fineness and the dispersion stability of the dispersed particles, collagen derivatives are particularly preferred among others.

The content of the water-soluble polymers in the dispersion composition of the present invention is preferably in the range of from 0.001% by mass to 5% by mass, and more preferably in the range of from 0.01% by mass to 1% by mass.

The content of the water-soluble polymers in the composition is, from the viewpoint of dispersion stability,

4

preferably in the range of from 0.1 times to 10 times, and more preferably in the range of from 0.5 times to 5.0 times the total mass of the poorly water-soluble polyphenol compounds in the composition.

[Emulsifier]

The emulsifier of the present invention preferably has, from the viewpoint of the emulsifying power, an HLB of 10 or higher, more preferably 12 or higher. When the HLB is too low, the emulsifying power may be insufficient.

Here, the HLB represents a hydrophilicity-hydrophobicity balance which is usually used in the field of surfactants, and a formula which is usually used, for example, Kawakami formula or the like can be employed. Kawakami formula is shown below:

$$HLB = 7 + 11.7 \log (M_w / M_o).$$

Here, $M_w$ represents a molecular weight of the hydrophilic group and $M_o$ represents a molecular weight of the hydrophobic group.

Values of HLB described in catalogues or the like may be employed.

As can be also seen from the above formula, by taking advantage of the additive property of the HLB, an emulsifier having an arbitrary HLB value can be obtained.

The dispersion composition of the present invention contains a sucrose fatty acid ester as an emulsifier.

The sucrose fatty acid ester used in the present invention is preferably a sucrose fatty acid ester in which the number of carbons of the fatty acid which is used in the formation of the sucrose fatty acid ester is 12 to 20, more preferably 14 to 16, and most preferably 14. When the number of carbons of the fatty acid is 12 or higher, a sufficient emulsion stability is easily obtained even in an emulsion composition which does not contains a glycerin fatty acid ester as described below, and on the other hand, by making the number of carbons of the fatty acid be 18 or smaller, the dispersion stability of the poorly water-soluble polyphenol compound can be effectively improved, which are preferred individually.

Preferred examples of the sucrose fatty acid ester of the present invention include sucrose monooleate, sucrose monostearate, sucrose monopalmitate, sucrose monomyristate and sucrose monolaurate. In the present invention, these sucrose fatty acid esters can be used alone or in combination.

Examples of commercially available sucrose fatty acid esters include RYOTO SUGAR ESTERs S-1170, S-1170F, S-1570, S-1670, P-1570, P-1670, M-1695, O-1570, OWA-1570, L-1695 and LWA-1570 manufactured by Mitsubishi-Kagaku Foods Corporation; and DK ESTERr SS, F160, F140, F110 and F90, and COSMELIKEs S-110, S-160, S-190, P-160, M-160, L-160, L-150A, L-160A and 0-150 manufactured by DAI-ICHI KOGYO SEIYAKU CO., LTD..

In the present invention, the content of the above sucrose fatty acid ester is preferably 20 to 85% by mass, and more preferably from 25 to 75% by mass, based on the mass of the total solid content of the dispersion composition. When the content of the sucrose fatty acid esters is 20% by mass or higher, the dispersion stability can be favorable, and a dispersion composition having a small particle diameter can be effectively obtained. When the content of the sucrose fatty acid esters is 85% by mass or lower, the foaming of the dispersion composition can be appropriately restrained.

In the dispersion composition of the present invention, the content of the sucrose fatty acid esters is preferably from 0.1 times to 10 times, and still more preferably from 0.5 times to 5.0 times the total mass of the poorly water-soluble polyphenol compound in the composition.

In the dispersion composition of the present invention, additional emulsifier(s) may be used in combination with the sucrose fatty acid ester. The content of the emulsifiers in the dispersion composition of the present invention can be, in general, from 10% by mass to 90% by mass and preferably, from the viewpoint of dispersion stability, from 25% by mass to 50% by mass, based on the total mass of the oil components of the dispersion composition. When the additional emulsifier is used in combination with the sucrose fatty acid ester, the total amount of the additional emulsifiers and the sucrose fatty acid esters may be in the range above. In this case, in order to surely obtain the effect of the present invention, the content ratio of the additional emulsifiers to the total amount of the emulsifiers is preferably 50% by mass or lower, and more preferably 30% by mass or lower.

The additional emulsifier which can be used in combination with the sucrose fatty acid ester is not particularly restricted as long as the additional emulsifier dissolves in an aqueous medium, and a noninonic emulsifier is preferred because of the low irritancy, a small effect on the environment or the like. Examples of the nonionic emulsifier include organic acid monoglyceride, propylene glycol fatty acid ester, polyglycerin condensed ricinoleate, sorbitan fatty acid ester and polyoxyethylene sorbitan fatty acid ester.

In the dispersion composition of the present invention, among the above emulsifiers, the total content of glycerin fatty acid esters and polyglycerin fatty acid esters (these are herein generally referred to as "glycerin fatty acid ester(s)") in the composition is 0, or 0.1 or fewer times the total mass of sucrose fatty acid esters in the composition. As described above, when the content of glycerin fatty acid esters is 0.1 times or less the total mass of sucrose fatty acid esters, the dispersion stability of the dispersion composition including a poorly water-soluble polyphenol compound can be favorable.

The mass ratio of the glycerin fatty acid esters is 0.1 or fewer times, and preferably, from the viewpoint of surely preventing the aggregation of the polyphenol compound, 0.05 or fewer times, more preferably 0.001 or fewer times, and most preferably 0, that is, no glycerin fatty acid ester.

The sorbitan fatty acid ester which can be used in combination with the sucrose fatty acid ester is preferably a sorbitan fatty acid ester having 8 or more carbon atoms in the fatty acid, and more preferably a sorbitan fatty acid ester having 12 or more carbon atoms in the fatty acid. Preferred examples of the sorbitan fatty acid ester include sorbitan monocaprylate, sorbitan monolaurate, sorbitan monostearate, sorbitan sesquistearate, sorbitan tristearate, sorbitan isostearate, sorbitan sesquiisostearate, sorbitan oleate, sorbitan sesquioleate and sorbitan trioleate.

In the present invention, these sorbitan fatty acid esters can be used alone or two or more thereof can be used in combination.

Examples of commercially available sorbitan fatty acid ester include NIKKOLs SL-10, SP-10V, SS-10V, SS-10MV, SS-15V, SS-30V, SI-10RV, SI-15RY, SO-10V, SO-15MV, SO-15V, SO-30V, SO-10R, SO-15R, SO-30R and SO-15EX manufactured by Nikko Chemicals Co., Ltd.; SORGENs 30V, 40V, 50V, 90 and 110 manufactured by Dai-ichi Kogyo Seiyaku Co., Ltd.; and RHEODOLs AS-10V, AO-10V, AO-15V, SP-L10, SP-P10, SP-S10V, SP-S30V, SP-O10V and SP-O30V manufactured by Kao Corporation.

Polyoxyethylene sorbitan fatty acid ester is preferably those having 8 or more carbon atoms in the fatty acid, and more preferably 12 or more carbon atoms. The length of the ethylene oxide in the polyoxyethylene (the mole number of added ethylene oxide) is preferably from 2 to 100, and more preferably from 4 to 50.

Preferred example of the polyoxyethylene sorbitan fatty acid ester include polyoxyethylene sorbitan monocaprylate, polyoxyethylene sorbitan monolaurate, polyoxyethylene sorbitan monostearate, polyoxyethylene sorbitan sesquistearate, polyoxyethylene sorbitan tristearate, polyoxyethylene sorbitan isostearate, polyoxyethylene sorbitan sesquiisostearate, polyoxyethylene sorbitan oleate, polyoxyethylene sorbitan sesquioleate and polyoxyethylene sorbitan trioleate.

These polyoxyethylene sorbitan fatty acid esters can be used alone or in combination.

Examples of commercially available polyoxyethylene sorbitan fatty acid esters include NIKKOL TL-10, NIKKOL TP-10V, NIKKOL TS-10V, NIKKOL TS-10MV, NIKKOL TS-106V, NIKKOL TS-30V, NIKKOL TI-10V, NIKKOL TO-10V, NIKKOL TO-10MV, NIKKOL TO-106V, NIKKOL TO-30V manufactured by Nikko Chemicals Co., Ltd.; RHEODOLs TW-L106, TW-L120, TW-P120, TW-S106V, TW-S120V, TW-S320V, TW-O106V, TW-O120V, TW-O320V, TW-1S399C, and RHEODOL SUPERs SP-L10 and TW-L120 manufactured by Kao Corporation; and SORGENs TW-20, TW-60V and TW-80V manufactured by Dai-ichi Kogyo Seiyaku Co., Ltd.

Further, as the emulsifier of the present invention, phospholipids such as lecithin may be contained. When a phospholipid is contained, from the viewpoint of the dispersion stability, the phospholipids may be contained by from 0.01 times to 0.3 times the total mass of the oil components contained in the oil phase.

Phospholipids which can be used in the present invention are those having a glycerin skeleton, and a fatty acid residue and a phosphate residue as essential structural components to which base(s), polyhydric alcohol(s) and/or the like are connected, which are also referred to as lecithin. Since phospholipids have a hydrophilic group and a hydrophobic group in the molecule, they are conventionally widely used as an emulsifier in the field of foods, pharmaceuticals and cosmetics.

Industrially, lecithin whose purity is 60% or higher is used and can also be used in the present invention. From the viewpoint of the formation of the fine particles of the oil drop and the stability of the functional oil components, those generally referred to as a high purity lecithin can be preferably used, whose purity is 80% or higher and more preferably 90% or higher.

Examples of phospholipids can include a variety of conventionally known ones which are obtained by extraction separation from the living body of plants, animals and microorganisms.

Specific examples of such phospholipids include a variety of lecithins derived from plants such as soybean, corn, peanut, rapeseed and barley; from animals such as yolk and cow; and from microorganisms such as E. coli.

Examples of the compound name of such lecithins include glycerolecithins such as phosphatidic acid, phosphatidylglycerol, phosphatidylinositol, phosphatidylethanolamine, phosphatidyl methyl ethanolamine, phosphatidylcholine, phosphatidylserine, bisphosphatidic acid, diphosphatidylglycerol (cardiolipin); and sphingolecithins such as sphingomyelins.

In the present invention, other than the high purity lecithins, hydrogenated lecithins, enzyme-decomposed lecithins, enzyme-decomposed hydrogenated lecithins, hydroxy lecithins and the like can be used. These lecithins which can be used in the present invention can be used alone or plural types thereof can be used in the form of a mixture.

[Other oil components]

When the dispersion composition of the present invention is used for food applications, cosmetic applications or pharmaceutical applications, the dispersion composition can contain functional materials for foods, functional materials for cosmetics or functional materials for pharmaceuticals depending on each of the applications as other oil components.

The term "functional component" for each of the applications in the present invention means an oil component in which a prescribed physiological effect can be expected to be induced on a living body when the functional component is applied to the living body and when the functional component is applied as a part of foods, cosmetics or pharmaceuticals. The above-mentioned poorly water-soluble polyphenol compound may correspond to such a functional material.

The oil component of the present invention means an component which is generally recognized as an oil component in the fields of cosmetics, pharmaceuticals and foods. These oil components can constitute a part of the dispersed particles in the dispersion composition of the present invention.

Examples of other oil components which can be used in the present invention include ceramides such as ceramides, sphingo glycolipids, sphingosines and phytosphingosines; stenones; sterols such as beta sitosterols, stigmasterols and ursolic acid; carotenoids such as carotenes and astaxanthins; oils and fats such as coconut oils, ubiquinones; fat soluble vitamins such as tocopherol, tocotrienol and retinoids, and other various compounds which are known to be used for intended applications. These may be used alone or two or more thereof may be used in combination.

In the dispersion composition of the present invention, the total content of such other oil components which are used is, from the viewpoint of the particle size of the dispersed particles and the emulsion stability in view of the application for pharmaceuticals and cosmetics, preferably from 0.1% by mass to 50% by mass, more preferably from 0.2% by mass to 25% by mass and still more preferably from 0.5% by mass to 10% by mass based on the total mass of the dispersion.

When the content of the oil components is 0.1% by mass or higher as described above, the effect of the active components can be sufficiently exerted, which makes easy to apply the dispersion composition to pharmaceuticals and cosmetics. On the other hand, when the content of the oil components is 50% by mass or less, an increase in the particle size of the dispersed particles and deterioration of emulsion stability are restrained, thereby obtaining a stable composition.

Polyhydric alcohol

The dispersion composition of the present invention can further contain a polyhydric alcohol. Examples of the polyhydric alcohol include glycerin, 1,3-butanediol, and ethylene glycol. Examples of the polyhydric alcohol further include polysaccharides such as reduced starch syrup, sucrose, erythritol, xylitol, glucose, galactose, sorbitol, maltotriose and trehalose. These can be used alone, or two or more thereof can be used in combination.

The content of the polyhydric alcohols based on the total mass of the dispersion composition is, from the viewpoint of the dispersion stability and the storage stability and the viscosities of the dispersion and the composition, preferably from 5 to 60% by mass, more preferably from 5 to 55% by mass and still more preferably from 5 to 50% by mass based on the total mass of the dispersion composition.

When the content of the polyhydric alcohols is 5% by mass or higher, a sufficient storage stability is easily obtained due also to the type, the content or the like of the oil components, which is preferable. On the other hand, when the content of the polyhydric alcohols is 60% by mass or lower, the maximum effect is obtained, and an increase in the viscosity of the dispersion composition is easily restrained, which is preferable.

[Other additives]

In the dispersion composition of the present invention, as long as the effect of the present invention is not deteriorated, other additives usually used for the individual applications such as a variety of medicinal properties, preservatives and colorants can be additionally used depending on the applications of the dispersion composition of the present invention.

For example, in the case that the dispersion composition of the present invention is used for an external composition such as a skin external preparation, examples of other components include moisturizing agents such as glycine betaine, xylitol, trehalose, urea, neutral amino acid and basic amino acid; therapeutic agents such as allantoin; organic powders such as cellulose powder, nylon powder, cross-linked silicone powder, cross-linked methylpolysiloxane, porous cellulose powder and porous nylon powder; inorganic powders such as anhydrous silica, zinc oxide and titanium oxide; refrigerants such as menthol and camphor; as well as plant extracts; pH buffers; anti-oxidants; ultraviolet absorbers; preservatives; perfumes; bactericides; and pigments.

The pH of the dispersion composition of the present invention is from 6 to 8 and preferably from 6.5 to 7.5. By making the pH of the dispersion composition be in the above range, the dispersion composition shows a favorable dispersion stability and storage stability. In order to adjust the pH of the dispersion composition, a variety of pH adjusters may be used.

In order for the pH of the dispersion composition to be in a prescribed range, the pH adjuster may be added and blended when an oil phase or a water phase is prepared, or may be added directly into the obtained dispersion composition. Examples of usable pH adjusters include acids such as hydrochloric acid and phosphoric acid and alkalis such as sodium hydroxide; a variety of inorganic salts usually used in the field; and buffer agents such as lactic acid/sodium lactate, citric acid/sodium citrate, succinic acid/sodium succinate.

[Water-soluble organic solvent]

The dispersion composition of the present invention may include a water-soluble organic solvent which is used in the below-described manufacturing process. This water-soluble organic solvent is not included in the "oil component" herein.

The water-soluble organic solvent in the present invention is preferably used in the below-described method for manufacturing a dispersion composition in order to prepare an oil phase by mixing oil phase components, and the water-soluble organic solvent is preferably removed after mixing with a water phase.

The water-soluble organic solvent used in the present invention refers to an organic solvent having a solubility to water at 25°C of 10% by mass or higher. From the viewpoint of the stability of the finished dispersion, the solubility to water is preferably 30% by mass or higher and more preferably 50% by mass or higher.

The water-soluble organic solvent can be used alone or plural water-soluble organic solvents can be used as a mixed solvent. The water-soluble organic solvent can be also used as a mixture with water. When the mixture with water is used, the above water-soluble organic solvent is preferably contained by at least 50% by volume or more and more preferably 70% by volume or more.

Examples of such water-soluble organic solvents include methanol, ethanol, 1-propanol, 2-propanol, 2-butanol, acetone, tetrahydrofuran, acetonitrile, methyl ethyl ketone, dipropylene glycol monomethyl ether, methyl acetate, methyl acetoacetate, N-methyl pyrrolidone, dimethyl sulfoxide, ethylene glycol, 1,3butanediol, 1,4butanediol, propylene glycol, diethylene glycol, triethylene glycol and mixture thereof. Among these, ethanol, propylene glycol or acetone is preferred, and ethanol or a mixed liquid of ethanol and water is particularly preferred when the application is limited to foods.

-Particle size-

The average volume particle size of the dispersed particles in the dispersion composition of the present invention is from 1 nm to 200nm, preferably from 1 nm to 75 nm, more preferably from 1 mn to 50 nm and most preferably from 1 nm to 30 nm.

By making the particle size of the dispersed particle size be 1 nm to 200 nm, the transparency of the dispersion composition can be secured, and when the dispersion composition of the present invention is used for, for example, cosmetics, pharmaceuticals, foods or the like, the transparency of the dispersion composition is secured and desired effects such as skin absorption can also be favorably exerted.

The particle size of the dispersed particles can be measured by a commercially available particle size distribution meter.

As a particle size measuring method, optical microscopy, confocal laser scanning microscopy, electron microscopy, atomic force microscopy, a static light scattering method, laser diffractometry, a dynamic light scattering method, centrifuge, anelectric pulse mensuring method, a chromatography method, an ultrasonic attenuation method and the like are known, and apparatuses using corresponding principles are commercially available.

In the measurement of the particle size of the dispersed particles of the present invention, a dynamic light scattering method is preferably applied because of the range of particle size and the easiness of the measurement.

Examples of the commercially available measuring apparatuses using a dynamic light scattering method include Nano Truck UPA (Nikkiso Co., Ltd.), Dynamic Light Scattering Particle Size Analyzer LB-550 (Horiba, Ltd.)., Fiber-Optics Particle Size Analizer FPAR-1000 (Otsuka Electronics Co. Ltd.)

The value of the particle size of the dispersed particle of the present invention is measured by using Dynamic Light Scattering Particle Size Analyzer LB-550 (Horiba, Ltd.)., and specifically, values measured in the following manner are employed.

That is, in the method for measuring the particle size, a sample is diluted with pure water such that the concentration of the oil components contained in the sample aliquoted from the dispersion composition of the present invention is 1% by mass and the measurement is carried out by using a quartz cell. The particle size can be determined as the median diameter by setting the refraction index of the sample to 1.600; the refraction index of the dispersion medium to 1.333 (pure water); and the viscosity of the dispersion medium to the viscosity of pure water.

When dispersed particles are used for an oil phase in combination with other oil components in the dispersion composition of the present invention, oil phase particles can be obtained in which the particle size of the dispersed particles contained as the oil phase is miniaturized down to 200 nm or smaller, which is desired, due to a factor of the components contained in the dispersion composition, as well as due to factors such as stirring conditions (shear force, temperature and pressure) in the below-described method for manufacturing a dispersion composition, use conditions of a micromixer and the ratio of an oil phase to a water phase.

<Method for manufacturing dispersion composition>

The dispersion composition of the present invention is obtained by a manufacturing method including: preparing an oil phase by dissolving one or more oil phase components including the poorly water-soluble polyphenol in a good solvent for the poorly water-soluble polyphenol (an oil phase preparation process); and mixing the obtained oil phase and a phase of a poor solvent for the poorly water-soluble polyphenol compound to obtain the dispersion composition which includes the poorly water-soluble polyphenol compound and includes a dispersed particle having an average volume particle size of 200 nm or smaller (a mixing process).

The good solvent for the poorly water-soluble polyphenol compound used when an oil phase is prepared is a solvent which is in a form of a liquid at room temperature and which can dissolve at least 0.1% by mass or more of a poorly water-soluble polyphenol compound at 25°C. Examples of such a good solvent for a poorly water-soluble polyphenol compound can include a water-soluble organic solvent or an aqueous alkali solution.

Examples of a water-soluble organic solvent as a good solvent for a poorly water-soluble polyphenol compound include those described above as they are.

An aqueous alkali solution as a good solvent for a poorly water-soluble polyphenol compound can be selected appropriately depending on the type of the poorly water-soluble polyphenol compound or the amount of the poorly water-soluble polyphenol compound added, and can be adjusted to be an aqueous solution showing a pH of from 10 to 12 by a strong base such as NaOH. When the aqueous solution has a pH of 10 or higher, a sufficient difference of solubilities between the solution and the below-described poor solvent is generated and a poorly water-soluble polyphenol compound can be favorably dispersed; and when the aqueous solution has a pH of 12 or lower, the physical properties and function of the other components in the dispersion composition are not largely deteriorated.

The poor solvent in the present invention refers to a solvent in which a poorly water-soluble polyphenol compound is poorly soluble, that is, a poorly water-soluble polyphenol compound is hardly to dissolve or does not dissolve.

The term "water phase" is used herein as a term as opposed to the term "oil phase" irrespective of the type of the poor solvent. The water phase of the dispersion composition of the present invention may contain, as a water phase component, another component which dissolves in a poor solvent for a poorly water-soluble polyphenol compound, for example, water. Such water-soluble water phase components may contain a functional component which can show a specific function.

Preferable examples of such a poor solvent include aqueous media such as: water when the good solvent is a water soluble organic solvent; and acid aqueous solutions when the good solvent is an aqueous alkali solution.

The acid aqueous solutions used as a poor solvent can be appropriately selected depending on the type of the poorly water-soluble polyphenol compound or the amount of the poorly water-soluble polyphenol compound added, the pH of the aqueous alkali solution as a good solvent which is used and the like, and examples thereof also include strong acids and weak acids. For example, when an aqueous solution such as sodium dihydrogen phosphate showing a pH of from 3 to 7 is used, a pH of from about 6 to about 8 is obtained, which is preferable. When the aqueous solution has a pH of 7 or lower, a sufficient difference of solubilities between the solution and the aqueous alkali solution as a good solvent is generated and a poorly water-soluble polyphenol compound can be favorably dispersed; and when the aqueous solution has a pH of 3 or higher, the physical properties and function of the other components in the dispersion composition are not largely deteriorated. More preferable aqueous alkali solution can have a pH of from 4 to 5.

The mixing of water phase components and oil phase components may be carried out by using a known method such as a high-pressure emulsification method in which a shear force at 100 MPa or higher is added, or a jet injection method in which the oil phase components are directly injected into the water phase components. From the viewpoint of the particle size, the dispersion stability and the storage stability of the dispersed particles, the mixing is preferably carried out by using a method using a micromixer in which the oil components and the water phase components are individually passed through a micro flow channel, whose sectional area at the narrowest portion is from 1 $\mu m^2$ to 1 $mm^2$, and then the phases are combined and mixed.

Here, the viscosity of the water phase is preferably 30 mPa·s or lower from the viewpoint of the fineness of the dispersed particles.

In the present invention, the temperature at the time when the oil phase components and the water phase components are mixed is preferably 40°C or lower. The temperature of 40°C or lower at the time of mixing may be attained when the oil phase components and the water phase components are mixed, and the set temperature range can be changed as required according to a mixing (emulsifying) method which is applied. In a method using a micromixer, a temperature at least in the range before mixing and immediately after mixing may be 40°C or lower. For example, the temperature is determined based on each of the temperatures of the water phase components and the oil components just before mixing and the temperature measured immediately after dispersion. From the viewpoint of temporal stability of the dispersion composition, it is preferred that the temperature of the dispersion during mixing be 35°C or lower.

A method for manufacturing a dispersion composition of the present invention include; for example, the following steps:

a) preparing a water phase using a poor solvent for a poorly water-soluble polyphenol compound (water or the like),
b) preparing an oil phase using oil phase components including at least a poorly water-soluble polyphenol compound, and
c) a step in which the oil phase and the water phase are mixed to be dispersed by using a micromixer in the method described below in detail, to obtain a dispersion composition including dispersed particles having an average volume particle size of from 1 nm to 1.00 nm.

The ratio (mass) of the oil phase to the water phase in the emulsification and dispersion is not particularly restricted, and preferably from 0.1/ 99.9 to 50/ 50, more preferably from 0.5/ 99.5 to 30/ 70 and still more preferably from 1/ 99 to 20/ 80 in terms of oil phase/ water phase ratio (% by mass).

By setting the oil phase/ water phase ratio to the above range, active components can be sufficiently contained and a practically sufficient emulsion stability can be obtained, which are preferable.

When a composition in a powder form is desired to be obtained by using a dispersion composition of the present invention, the composition in a powder form can be obtained by the addition of a step in which the dispersion composition in a form of an emulsion obtained above is dried by, for example, spray drying.

The components contained in the oil phase and the water phase in the method for manufacturing a dispersion composition are the same as the constituents of the above-mentioned dispersion composition of the present invention. Preferred examples and preferred amount of the components are also the same as that of the above-mentioned dispersion composition and the preferable combinations thereof are more preferred.

[Micromixer]

In the manufacturing method applied for manufacturing a dispersion composition of the present invention, in order to stably form dispersed particles of from 1 nm to 100 nm, it is preferable to use a manufacturing method in which the oil phase components and the water phase components are individually passed through a micro flow channel, whose sectional area at the narrowest portion is from 1 $\mu m^2$ to 1 $mm^2$, and then the phases are combined to be mixed.

From the viewpoint of obtaining fine dispersed particles, the mixing of the oil phase and the water phase is preferably a mixing by a counter collision.

The most appropriate apparatus in which the mixing is performed by a counter collision is a counter-collision micromixer. A micromixer mainly mixes two different liquids in a minimal space. One liquid is an organic solvent phase including a functional oil component, and the other liquid is a water phase which is an aqueous solution.

When a micromixer is applied to the preparation of an emulsion having a small particle size which is one of micro chemical processes, a favorable dispersion is easily obtained in which relatively low energy is required and heat generation is small, the particle sizes are more uniform and a better storage stability is obtained compared with the case of using a normal stirring/emulsifying/dispersing method or a high pressure homogenizer emulsion dispersion.

The method of dispersing using a micromixer is, in brief, separating a water phase and an oil phase into minimal spaces respectively, and each of the minimal spaces are allowed to be in contact, or to collide with each other. This method is clearly different from a membrane emulsification method or a microchannel emulsification method in which only one of the water phase and the oil phase is separated into minimal spaces and the other phase is in bulk. Practically, when only one of the water phase and the oil phase is separated into minimal spaces, the effect of the present invention is not obtained. There are known micromixers having a variety of structures. Focusing on the flow and the mixing in a microchannel, there are two types of methods: a method of mixing by maintaining a laminar flow; and a method of mixing by disturbing a flow, that is by using a turbulence. In the method of mixing by maintaining a laminar flow, by making the size of the depth of the flow channel larger than the size of the width of the flow channel, the area of interface between two liquids becomes as large as possible and the thicknesses of both of the layers becomes thin, thereby improving the efficiency of mixing. A method is also designed in which inlets of two liquids are divided and the liquids are flowed alternately to make a multilayer flow.

On the other hand, as the method of mixing using a turbulence, a method in which each of the liquids are separated into narrow flow channels and flowed relatively high speed is generally used. A method is also designed in which, by using arrayed micronozzles, the one liquid is jetted into the other liquid which is introduced into a minimal space. A method in which liquids flowing at a high speed are forced to be in contact with each other particularly has a favorable mixing effect. In the former method using a laminar flow, in general, the obtained particles are large and have a relatively uniform distribution. In the latter method using a turbulence, a very fine emulsion may be obtained and the method using a turbulence may be a favorable method in view of the stability and the transparency in many cases. Representative examples of methods using a turbulence include a comb-type and a collision-type. As typified by those manufactured by IMM mixer, the comb-type micromixer has a structure in which two comb-shaped flow channels are disposed such that the two comb-shaped flow channels are opposed to each other and alternately indented.

A collision-type micromixer as typified by a KM mixer has a structure in which a forced contact is carried out by using a kinetic energy. Specifically, a central collision type micromixer disclosed in Nagasawa et al ("H. Nagasawa et al., Chem. Eng. Technol, 28, No.3, 324-330 (2005)" and JP 2005-288254 A) is exemplified. In the method in which a water phase and an organic solvent phase countercollide with each other, the mixing time is extremely short and an oil phase droplet is formed instantaneously, whereby an emulsion or a dispersion, which has an extremely small particle size, is easily formed.

In the present invention, when emulsification is carried out by micromixing using a collision type-micromixer, regarding the temperature at the time of emulsification (emulsification temperature), from the viewpoint of uniformity of the particle size of the obtained emulsion, micromixing is carry out under the conditions in which the temperature of the other minimal space of the micromixer (the temperature of the micromixing portion of the micromixer) is preferably 80°C or lower, more preferably from 0°C to 80°C and particularly preferably from 5°C to 75°C. When the emulsification temperature is 0°C or higher, he emulsification temperature can be controlled because the dispersion medium is mainly water, which is preferable. The retained temperature of the minimal space of the micromixer is preferably 100°C or lower. When the retention temperature is 1.00°C or lower, the retention temperature can be easily controlled, and a micro-bumping phenomenon which adversely influences the emulsification performance can be prevented. It is more preferable to control the retention temperature at a temperature of 80°C or lower.

The retention temperatures of the oil phase and the poor solvent phase of the micromixer which are separated into minimal spaces, and the minimal spaces of the micromixer vary depending on the components contained in the poor solvent phase and the oil phase, and are each individually preferably from 0°C to 50°C and particularly preferably from 5°C to 25°C. The retention temperature of the minimal spaces of the micromixer, the retention temperature of the oil phase and the poor solvent phase which is separated into the minimal spaces of the micromixer and the retention temperature of the oil phase and the poor solvent phase before the phases are separated into the minimal spaces of the micromixer (that is, the retention temperature of a supply tank of the oil phase and the poor solvent phase) may be different from each other, and however, in view of the stability of mixing, these temperatures are preferably the same.

In the present invention, emulsification is particularly preferably carried out by micromixing under the conditions in which the retention temperatures of the water phase and the oil phase before and after the phases are separated into minimal spaces of the micromixer, and the minimal spaces and the other minimal spaces of the micromixer are higher than room temperature, and then an oil-in-water emulsion obtained by the micromixer is collected and cooled to room temperature.

The sectional areas of the minimal spaces (flow channels) of the micromixer of the present invention at the narrowest portion are from 1 $\mu m^2$ to 1 $mm^2$, and, from the viewpoint of small particle size of emulsion and the sharpness of the particle size distribution, preferably from 500 $\mu m^2$ to 50,000 $\mu m^2$.

The sectional areas of the minimal spaces (flow channels) of the micromixer for use in the water phase of the present invention at the narrowest portion are, from the viewpoint of the stability of mixing, particularly preferably from 1,000 $\mu m^2$ to 50,000 $\mu m^2$.

The sectional areas of the minimal spaces (flow channels) of the micromixer for use in the oil phase at the narrowest portion are, from the viewpoint of small particle size of emulsion and the sharpness of the particle size distribution, particularly preferably from 500 $\mu m^2$ to 20,000 $\mu m^2$.

When mixing (emulsion dispersion) is carried out by a micromixer, the flow rates of the oil phase and the water phase at the time of emulsion dispersion vary depending on the micromixer, which is used. From the viewpoint of small particle size of the emulsion and the sharpness of the particle size distribution, the flow rate of the water phase is preferably from 10 ml/ min to 500 ml/ min, more preferably from 20 ml/ min to 350 ml/ min and particularly preferably from 50 ml/ min to 200 ml/ min.

From the viewpoint of small particle size of the emulsion and the sharpness of the particle size distribution, the flow rate of the oil phase is preferably from 1 ml/ min to 100 ml/ min, more preferably from 3 ml/ min to 50 ml/ min and particularly preferably from 5 ml/ min to 50 ml/ min.

The values of the flow rates of the both phases divided by the sectional areas of the microchannels, that is, the flow rate ratios (Vo/Vw) of the both phases are, in view of the fineness of the particles and the design of the micromixer, preferably in the range of from 0.05 to 5. Here, Vo represents the flow rate of the organic solvent phase containing a water-insoluble natural component and Vw represents the flow rate of the water phase. The flow rate ratio (Vo/Vw) is, from the viewpoint of the further fineness of the particles, most preferably in the range of from 0.1 to 3.

The liquid sending pressures of the water phase and the oil phase are preferably from 0.030 MPa to 5 MPa and from 0.010 MPa to 1 MPa, more preferably from 0.1 MPa to 2 MPa and from 0.02 MPa to 0.5 MPa and particularly preferably from 0.2 MPa to 1 MPa and from 0.04 MPa to 0.2 MPa, respectively. When the liquid sending pressures of the water phase is from 0.030 MPa to 5 MPa, a stable liquid sending flow rate tends to be maintained, and when the liquid sending pressures of the oil phase is from 0.010 MPa to 1 MPa, a uniform mixing property tends to be obtained, which is preferable.

In the present invention, regarding the flow rate, the liquid sending pressure and the retention temperature, a combination of respective preferable examples thereof is more preferred.

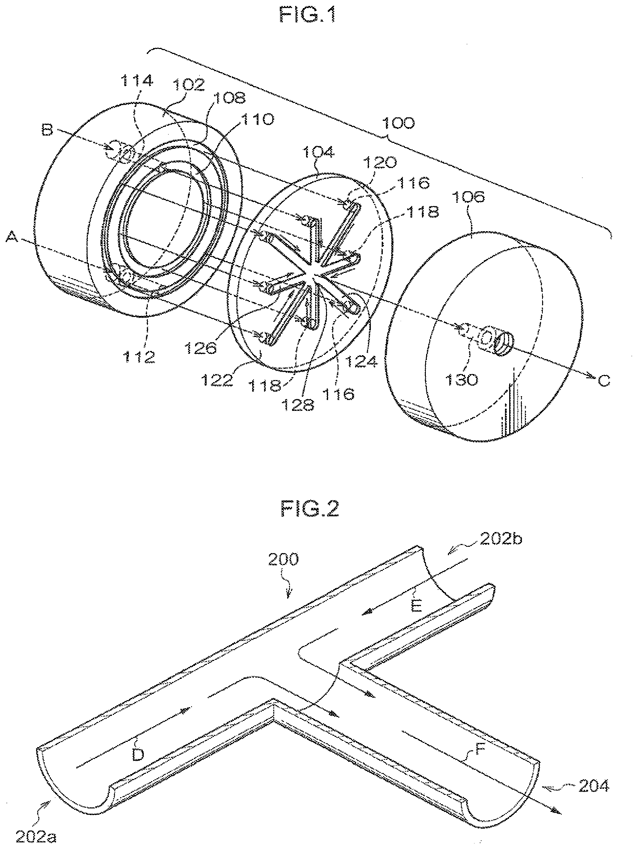

Next, a route in which the water phase and the oil phase are introduced into a micromixer and ejected as an oil-in-water emulsion is explained by using the example of a microdevice (Fig. 1) as one example of the micromixer of the present invention.

As shown in Fig. 1, in a microdevice 100, a supply element 102, a joining element 104 and discharge element 106, each having a cylindrical form, are formed.

On the surface of the supply element 102 opposing the joining element 104, ring-shaped channels 108 and 110 each having a rectangular cross section are formed concentrically as flow channels of the oil phase and the water phase of the present invention. In the supply element 102, bores 112 and 114 which penetrate in the thickness (or height) direction and reach ring-shaped channels respectively are formed.

In the joining element 104, a bore 116 which penetrates in the thickness direction is formed. The bore 116 is, when elements are engaged in order to form the microdevice 100, formed such that an end 120 of the bore 116 which is positioned at the surface of the joining element 104 opposing the supply element 102 is open at the ring-shaped channel 108. In the embodiment as shown in the figure, four bores 116 are formed, which are arranged at regular intervals in the circumferential direction of the ring-shaped channel 108.

In the joining element 104, a bore 118 is penetrated to be formed in the same manner as the bore 116. The bore 118 is also formed such that the bore is open at the ring-shaped channel 110 in the same manner as the bore 116. The bores 118 are also arranged at regular intervals in the circumferential direction of the ring-shaped channel 110 and arranged such that the bores 116 and the bores 118 are positioned alternately.

On the surface 122 of the joining element 104 opposing the discharge element 106, microchannel 124 and 126 are formed. One end of the microchannel 124 or 126 is an opening of the bore 116 or 118, and another end thereof is a center 128 of the surface 122. All microchannels extend from the bores toward the center 128 and are joined at the center. The cross section of the microchannel may, for example, be a rectangle.

In the discharge element 106, a bore 130 which penetrates in the thickness direction passing through the center is formed. This bore, therefore, is open at the center 128 of the joining element 104 on one end, and open outside the microdevice on the other end.

In the microdevice 100, fluids A and B supplied from outside of the microdevice 100 to the bores 112 and 114 pass through bores 112 and 114 and flow into the ring-shaped channel 108 and 110 respectively.

The ring-shaped channel 108 communicates with the bore 116 and the fluid A flowed into the ring-shaped channel 108 passes through the bore 116 and enters the microchannel 124. The ring-shaped channel 110 communicates with the bore 118 and the fluid B flowed into the ring-shaped channel 110 passes through the bore 118 and enters the microchannel 126. The fluids A and B flow in the microchannels 124 and 126 respectively, and then, flow toward the center 128 and are joined.

The joined fluids pass through the bore 130 and are discharged outside the microdevice as a stream C.

Such a microdevice 100 can have the following specifications.

The cross-section shape, width/ depth/ diameter of the ring-shaped channel 108: rectangular, 1.5/ 1.5/ 25 mm
The cross-section shape, width, depth, diameter of the ring-shaped channel 110: rectangular, 1.5/ 1.5/ 20 mm
The diameter and length of the bore 112: 1.5/ 10 mm (circular cross-section)
The diameter and length of the bore 114: 1.5/ 10 mm (circular cross-section)
The diameter and length of the bore 116: 0.5/ 4 mm (circular cross-section)
The diameter and length of the bore 118: 0.5/ 4 mm (circular cross-section)
The cross-section shape, width, depth, length of the microchannel 124: rectangle, cross-section, 350 $\mu$m/ 100 $\mu$m/ 12.5 mm/ 35000 $\mu$m$^2$
The cross-section shape, width, depth, length of the microchannel 126: rectangle, cross-section, 50 $\mu$m/ 100 $\mu$m/ 10 mm/ 5000 $\mu$m$^2$
The diameter and length of the bore 130: 500 $\mu$m, 10 mm (circular cross-section)

The sizes of the microchannels (124 and 126 in Fig. 1) in which a water phase and an oil phase collide are defined in preferable ranges in relation to the flow rates of the water phase and the oil phase.

In the present invention, the micromixer shown in JP 2004-33901 A can also be preferably used.

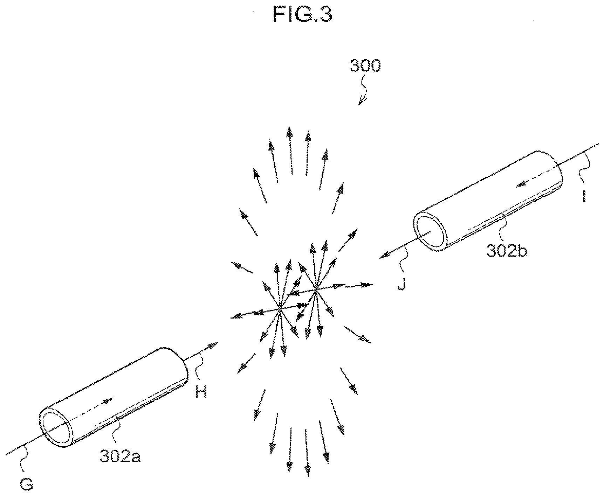

Fig. 2 is a schematic sectional view of a T-shaped microreactor showing one example of the mixing mechanism of a T-shaped microreactor. Fig. 3 is a conceptual diagram of a T-shaped microreactor showing one example of the mixing mechanism of a T-shaped microreactor.

Fig. 2 shows a cross section of the T-shaped flow channel 200 of a T-shaped microreactor. In the T-shaped flow channel 200, a fluid flowed from a flow inlet 202a in the direction of an arrow D direction and a fluid flowed from a flow inlet 202b in the direction of an arrow E direction collide at the center in the T-shaped flow channel 200 to be mixed, thereby obtaining fine fluid particles. The fine fluid particles flow out from a flow outlet 204 in the direction of an arrow F. This T-shaped microreactor is useful for mixing when the volume of the flow channel is small.

Fig. 3 shows a fluid mixing mechanism (concept) of another T-shaped microreactor 300. In the fluid mixing mechanism shown in Fig. 3, fluids flowed out from two flow channels 302a and 302b collide each other and are mixed to obtain fine fluid particles. That is, on the one hand, the fluid flows into the flow channel 302a in the direction of an arrow G and flows out in the direction of an arrow H. On the other hand, the fluid flows into the flow channel 302b in the direction of an arrow I and flows out in the direction of an arrow J. The fluids flowed out from the flow channels 302a and 302b respectively collide and are mixed to scatter in the directions approximately perpendicular to the directions of arrows G to J. Thus, in the fluid mixing mechanism described in Fig. 3, a fluid which is diffused by a method such as atomization is allowed to collide and to be mixed. By this collision and mixing, the fluid can become finer and a large contact surface can be obtained.

In the manufacturing method which can be applied to a dispersion composition of the present invention, the water-soluble organic solvent which is used is preferably removed after emulsification or dispersion through the microchannel. As a method of removing a solvent, evaporation methods using a rotary evaporator, a flash evaporator, an ultrasonic atomizer or the like, or membrane separation methods using an ultrafiltration membrane, a reverse osmotic membrane or the like are known, and an ultrafiltration membrane method is particularly preferred.

Ultra Filter (also referred to as UF in short) is an apparatus in which an undiluted liquid (a mixed aqueous solution of water, a high-molecular material, a low-molecular material, a colloidal substance and the like) is pressurized and water is added to the UF apparatus, thereby separating the undiluted liquid into two types of liquids which are a permeated liquid (low-molecular material) and a concentrated liquid (high-molecular material, colloidal material) to be taken out.

The ultrafiltration membrane is a typical asymmetric membrane manufactured by a Loeb-Sourirajan process. Examples of polymer material which is used include polyacrylonitrile, polyvinyl chloride-polyacrylonitrile copolymer, polysulfone, polyethersulfone, vinylidene fluoride, aromatic polyamide and cellulose acetate. Recently, a ceramic membrane is also used. In the ultrafiltration membrane, since, different from a reverse osmosis or the like, a pretreatment is not carried out, a fouling in which polymer is deposited on the membrane surface occurs. It is, therefore, usual that the membrane be washed with chemical agents or warm water periodically. For this reason, the membrane material is required to have resistance to chemical agents or heat resistance. Examples of the membrane module for the ultrafiltration membrane include a flat membrane, a tube-shaped membrane, a hollow fiber membrane and a spiral membrane. The performance index of the ultrafiltration membrane is a molecular weight cut off, and a variety of membranes having a molecular weight cut off of from 1,000 to 300,000 are commercially available. Examples of the commercially available membrane module include, but not limited thereto, MICROZA UF (Asahi Kasei Chemicals Corporation) and capillary-type element NTU-3306 (Nitto Denko Corporation).

In order to remove a solvent from the obtained emulsion, from the viewpoint of the solvent tolerance, the material of the membrane is particularly preferably polysulfone, polyethersulfone or aromatic polyamide. As the form of the membrane module, a flat membrane is mainly used in a laboratory scale and a follow fiber membrane and a spiral membrane are used in an industrial scale. A hollow fiber membrane is particularly preferred. The molecular weight cut off varies depending on the type of active components and usually is in the range of from 5,000 to 100,000.

The operation temperature can be from 0°C to 80°C, and is particularly preferably from 10°C to 40°C considering the deterioration of the active component.

Examples of the laboratory-scale ultrafiltration membrane apparatuses include ADVANTEC-UHP (Advantec Corporation) and Flow-Type Lab Test Unit RUM-2 (Nitto Denko Corporation) each using a flat-type module. Industrially, a plant can be formed by combining membrane modules in a size and a number decided depending on the required capacity. As a bench-scale unit, RUW-5A (Nitto Denko Corporation) and the like are commercially available.

To a manufacturing method which can be applied to a dispersion composition of the present invention, a process of concentrating the obtained emulsion may be added after the solvent removal. As the concentrating process, the same method and apparatus as those used in an evaporation method, a filtration membrane method or the like can be used. An ultrafiltration membrane method is a preferable method also in the case of concentrating. It is preferred that the same membrane as in the solvent removal can be used. An ultrafiltration membrane having a different molecular weight cut off can also be used as required. By operating at a different temperature from the temperature used in the solvent removal, it is also possible to improve the efficiency of the concentration.

The dispersion composition (emulsion) obtained by the mixing using the micromixer is an oil-in-water emulsion. In the method for manufacturing a dispersion composition of the present invention, the average volume particle size (median diameter) of the dispersed particles is from 1 nm to 200 nm. From the viewpoint of the transparency of the obtained dispersion composition, the average volume particle size of the dispersed particles is more preferably from 1 nm to 100 nm.

The particle size of the dispersed particles obtained by the manufacturing method as explained above can be determined by a commercially available particle size distribution meter or the like, the detail of which is as described above.

Since the dispersion composition of the present invention is a dispersion composition which includes a poorly water-soluble polyphenol compound and has an excellent dispersion stability, the dispersion composition of the present invention can be preferably included in cosmetic compositions, food compositions and pharmaceutical compositions respectively. Thus, compositions applied for various uses which include a poorly water-soluble polyphenol compound and have an excellent dispersion stability can be provided.

According to the present invention, cosmetic compositions, food compositions and pharmaceutical compositions of the present invention include, as the material for each of the compositions, a poorly water-soluble polyphenol compound in combination with oil-soluble or water-soluble functional materials which are characteristic of respective applications. Those skilled in the art can appropriately select oil-soluble or water-soluble functional materials which are characteristic of each application, and can manufacture cosmetic compositions, food compositions and pharmaceutical compositions of the present invention according to the description of the present invention in such a way that the effect of the present invention is obtained.

EXAMPLES

In the following, the present invention is further concretely explained by way of Examples, and not limited to the following Examples as long as they depart from the scope of the present invention. Unless otherwise noted , "part" is based on mass.

<Preparation of dispersion composition>

[Example 1]

The components described in the below Table 1 were stirred at room temperature for 1 hour to prepare an oil phase liquid and an water phase liquid respectively.

The obtained water phase liquid (oil phase) and the water phase liquid (water phase) each were warmed to 25°C and micromixed (dispersed) (oil phase : water phase by volume is 1 : 7) by using a KM-type micromixer 100/100, which is a collision-type, to obtain a dispersion liquid at 25°C (pH: 7.5). The use conditions of the micromixer is as follows:

- Microchannel -

Oil phase microchannel

cross-section shape/width/ depth/ length = rectangle/ 70 µm/ 100 µm/ 10 mm

Water phase microchannels

cross-section shape/width/ depth/ length = rectangle/ 490 µm/ 100 µm/ 10 mm

- Flow rate -

A water phase was introduced into an outer ring at a flow rate of 56.0 ml/ min., and an oil phase was introduced into an inner ring at a flow rate of 8.0 ml/ min. to be micromixed.

By removing the solvent from a dispersion liquid 1 obtained by repeated adjustments by using a thin film evaporator (manufactured by OKAWARA CORPORATION: EVAPOR (CEP-lab)) until the concentration of ethanol is 0.1% by mass ar lower, the dispersion liquid was quadruply concentrated such that the concentration of the poorly water-soluble polyphenol compound be 0.5% by mass to obtain a dispersion composition having a pH of 7.5. The concentration of the poorly water-soluble polyphenol compound in the dispersion composition is based on the total mass of the dispersion composition.

[Examples 2 to 7, Comparative Examples 1 to 7]

A dispersion composition was obtained in the same manner as in Example 1 except that the oil phase components and the water phase components were changed as described in Table 1 or Table 2. All of the dispersion compositions have a pH of from 6.5 to 7.5.

In Table 1 and Table 2, resveratrol manufactured by La Gardonnenque Corporation, curcumin manufactured by Lion McCormick Corporation and rutin manufactured by Wako Pure Chemical Industries, Ltd. were used. Any of these poorly water-soluble polyphenol compounds have a solubility to water of 0.1% by mass or lower. Regarding the sucrose fatty acid ester, for sucrose myristate, Ryoto Sugar Ester M-1695 (HLB = 16) manufactured by Mitsubishi-Kagaku Foods Corporation; for decaglyceryl monooleate, NIKKOL Decaglyn 1-O (HLB = 12) manufactured by Nikko Chemical Co. were employed.

The molecular weight of PSK Gelatin (manufactured by Nippi, Inc.) was 100,000, the molecular weight of SCP-5000 (peptide collagen manufactured by Nitta Gelatin Inc.) was 5,000, the molecular weight of hyaluronic acid (manufactured by KIBUN FOODS, Inc.) was about 80,000 and the molecular weight of gum arabic was about 300,000.

<Evaluation>

1. Particle size of the dispersed particle

The particle sizes of the dispersed particles in the dispersion compositions in Examples 1 to 7 and Comparative Examples 1 to 7 immediately after the preparation were measured by using Dynamic Light Scattering Particle Size Analyzer LB-550 (Horiba, Ltd.). In the measurement of the particle size, the dispersion composition was diluted with pure water such that the concentration of the dispersed particles is 1% by mass and a quartz cell was used. The particle size was determined as a median diameter by setting the refraction index of the sample to 1.600; the refraction index of the dispersion medium to 1.333 (pure water); and the viscosity of the dispersion medium to the viscosity of pure water. The results are shown in Table 1 and Table 2.

2. Evaluation of the temporal stability of the dispersion composition

The evaluation of the temporal stability was carried out by visual observation to judge transparent or opaque.

The transparency of the dispersion composition immediately after dispersion was observed by visual observation to judge transparent or opaque. Further, the dispersion compositions were stored in a thermostat bath at 40°C for 1 week, then cooled to 25°C and judged in the same manner by visual observation again. The results are shown in Table 1 and Table 2.

3. Evaluation of stability

Depending of the magnitude of the change in the particle sizes and outer appearance evaluations between immediately after the preparation and after stored at 40°C for 1 week, the dispersion compositions were evaluated as "A" (good), "B" (fair) and "C" (failure). "A" means that the transparency and the particle size of 100 nm or smaller are maintained both immediately after the dispersion and after the storage in the above conditions. On the other hand, "B" means that the transparency and the particle size of 200 nm or smaller are maintained both immediately after the dispersion and after the storage in the above conditions. "A" or "B" is practically acceptable. "C" means not being practical. The results are shown in Table 1 and Table 2.

Table 1

| | | Ex. 1 | Ex. 2 | Ex. 3 | Ex. 4 | Ex. 5 | Ex. 6 | Ex. 7 |
|---|---|---|---|---|---|---|---|---|
| Oil phase | resveratrol | 1.0 | 1.0 | 1.0 | 1.0 | | | 1.0 |
| | curcumin | | | | | 1.0 | | |
| | rutin | | | | | | 1.0 | |
| | sucrose fatty acid ester (M-1695) | 1.0 | 1.0 | 4.0 | 4.0 | 8.0 | 6.0 | 1.0 |
| | polyglycerin fatty acid ester (Decaglyn 1-O) | | | | | | | 0.05 |
| | ethanol | 80 | 80 | 80 | 80 | 80 | 80 | 80 |
| Water phase | pure water | 718.6 | 718.6 | 717.9 | 718.6 | 717.9 | 717.9 | 718.6 |
| | PSK gelatin (manufacture by Nippi, Inc.) | 1.4 | 1.4 | | | 2.1 | 2.1 | 1.4 |
| | SCP-5000 (peptide collagen manufactured by Nitta Gelatin Inc.) | | | 1.4 | | | | |
| | hyaluronic acid (manufactured by KIBUN FOODS, Inc.) | | | | 2.1 | | | |
| | gum arabic | | | | | | | |
| | NaOH | trace amount | trace amount | - | - | trace amount | trace amount | trace amount |
| Stability | particle size immediately after dispersion (nm) | 38.6 | 76.1 | 104.9 | 119.4 | 68.9 | 38.2 | 39.2 |
| | outer appearance | transparent | transparent | transparent | transparent | transparent | transparent | transparent |
| | particle size after 40°C thermostat × 1 W (nm) | 41.5 | 82.5 | 189.3 | 195.9 | 158.4 | 142.6 | 44.5 |
| | outer appearance | transparent | transparent | transparent | transparent | transparent | transparent | transparent |
| | stability | A | A | B | B | B | B | A |

(part)

Table 2

| | | Comp. Ex. 1 | Comp. Ex. 2 | Comp. Ex. 3 | Comp. Ex. 4 | Comp. Ex. 5 | Comp. Ex. 6 | Comp. Ex. 7 |
|---|---|---|---|---|---|---|---|---|
| Oil phase | resveratrol | 1.0 | 1.0 | 1.0 | | | | |
| | curcumin | | | | 1.0 | 1.0 | | |
| | rutin | | | | | | 1.0 | 1.0 |
| | sucrose fatty acid ester (M-1695) | | | 1.0 | | | | |
| | polyglycerin fatty acid ester (Decaglyn 1-O) | 1.0 | 1.0 | | 4.0 | 4.0 | 1.0 | 1.0 |
| | ethanol | 80 | 80 | 80 | 80 | 80 | 80 | 80 |
| Water phase | pure water | 720 | 718.6 | 720 | 720 | 717.9 | 720 | 718.6 |
| | PSK gelatin (manufacture by Nippi, Inc.) | | 1.4 | | | 2.1 | | |
| | SCP-5000 (peptide collagen manufactured by Nitta Gelatin Inc.) | | | | | | | 1.4 |
| | hyaluronic acid (manufactured by KIBUN FOODS, Inc.) | | | | | | | |
| | gum arabic | | | | | | | |
| | NaOH | - | trace amount | - | - | trace amount | - | trace amount |
| Stability | particle size immediately after dispersion (nm) | 523.6 | 451.2 | 315.6 | 252.6 | 136.5 | 21.5 | 121.9 |
| | outer appearance | opaque | opaque | opaque | opaque | transparent | transparent | transparent |
| | particles size after 40°C thermostat × 1 W (nm) | separated | separated | separated | separated | separated | separated | separated |
| | outer appearance | - | - | - | - | - | - | - |
| | stability | C | C | C | C | C | C | C |
| (part) | | | | | | | | |

EP 2 412 431 B1

As is clear from Table 1 and Table 2, the dispersion composition of the Examples of the present invention including a poorly water-soluble polyphenol compound had a small particle size of the dispersed particles, and had an excellent dispersion stability and stability overtime.

## Claims

1. A dispersion composition comprising:

   a poorly water-soluble polyphenol compound;
   an emulsifier including a sucrose fatty acid ester; and
   a water-soluble polymer,
   wherein,
   a content of a polyglycerin fatty acid ester in the composition is 0, or 0.1 or fewer times a total mass of the sucrose fatty acid ester in the composition, and a particle size of dispersed particles including the poorly water-soluble polyphenol compound is 200 nm or smaller;
   the poorly water-soluble polyphenol compound is at least one selected from the group consisting of resveratrol, curcumin, rutin, ellagic acid and quercetin; and
   the water-soluble polymer is at least one selected from the group consisting of proteins and polysaccharides.

2. The dispersion composition according to claim 1, wherein the water-soluble polymer has a molecular weight of from 1,000 to 600,000.

3. The dispersion composition according to any one of claims 1 or 2, wherein the water-soluble polymer is a collagen derivative.

4. The dispersion composition according to any one of claims 1 to 3, wherein the water-soluble polymer is at least one selected from the group consisting of kappa carrageenan, dextran and hyaluronic acids.

5. The dispersion composition according to any one of claims 1 to 4, wherein the total content of the water-soluble polymer in the composition is from 0.1 times to 10 times a total mass of the poorly water-soluble polyphenol compound in the composition.

6. The dispersion composition according to any one of claims 1 to 5, wherein a total content of the sucrose fatty acid ester in the composition is from 0.1 times to 10 times a total mass of the poorly water-soluble polyphenol compound in the composition.

7. A cosmetic composition comprising the dispersion composition according to any one of claims 1 to 6.

8. A food composition comprising the dispersion composition according to any one of claims 1 to 6.

9. A pharmaceutical composition comprising the dispersion composition according to any one of claims 1 to 6.

10. A method for manufacturing the dispersion composition according to any one of claims 1 to 6, the method comprising:

    an oil phase preparation process in which an oil phase is prepared by dissolving one or more oil phase components including the poorly water-soluble polyphenol compound in a good solvent for the poorly water-soluble compound; and
    a mixing process in which the obtained oil phase and a phase of a poor solvent for the poorly water-soluble polyphenol compound are mixed to obtain the dispersion composition which includes the poorly water-soluble polyphenol compound and includes dispersed particles having an average volume particle size of 200 nm or smaller.

11. The method for manufacturing a dispersion composition according to claim 10, wherein the good solvent for the poorly water-soluble polyphenol compound is a water-soluble organic solvent or an aqueous alkali solution.

12. The method for manufacturing a dispersion composition according to claim 10 or 11, wherein the mixing of the oil phase and the phase of the poor solvent is performed by allowing the oil phase and the phase of the poor solvent to pass through microchannels of 1 $\mu m^2$ to 1 $mm^2$ individually, and then combining and mixing the oil phase and the phase of the poor solvent.

13. The method for manufacturing a dispersion composition according to any one of claims 10 to 12, wherein the mixing is carried out by counter collision.

**Patentansprüche**

1. Dispersionszusammensetzung, umfassend:

eine schlecht wasserlösliche Polyphenolverbindung;
einen Emulgator, der einen Saccharosefettsäureester umfasst; und
ein wasserlösliches Polymer, wobei
der Gehalt an Polyglycerinfettsäureester in der Zusammensetzung gleich 0 ist, oder das 0,1-fache oder weniger der Gesamtmasse des Saccharosefettsäureesters in der Zusammensetzung beträgt und die Teilchengröße der dispergierten Teilchen, einschließlich der schlecht wasserlöslichen Polyphenolverbindung, 200 nm oder kleiner ist;
die schlecht wasserlösliche Polyphenolverbindung mindestens eine ist, die ausgewählt ist aus der Gruppe bestehend aus Resveratrol, Curcumin, Rutin, Ellagsäure und Quercetin; und
das wasserlösliche Polymer mindestens eines ist, das ausgewählt ist aus der Gruppe bestehend aus Proteinen und Polysacchariden.

2. Dispersionszusammensetzung gemäß Anspruch 1, wobei das wasserlösliche Polymer ein Molekulargewicht von 1.000 bis 600.000 aufweist.

3. Dispersionszusammensetzung gemäß einem der Ansprüche 1 oder 2, wobei das wasserlösliche Polymer ein Kollagenderivat ist.

4. Dispersionszusammensetzung gemäß einem der Ansprüche 1 bis 3, wobei das wasserlösliche Polymer mindestens eines ist, das ausgewählt ist aus der Gruppe bestehend aus Kappa-Carrageen, Dextran und Hyaluronsäuren.

5. Dispersionszusammensetzung gemäß einem der Ansprüche 1 bis 4, wobei der Gesamtgehalt des wasserlöslichen Polymers in der Zusammensetzung das 0,1-fache bis 10-fache der Gesamtmasse der schlecht wasserlöslichen Polyphenolverbindung in der Zusammensetzung beträgt.

6. Dispersionszusammensetzung gemäß einem der Ansprüche 1 bis 5, wobei der Gesamtgehalt des Saccharosefettsäureesters in der Zusammensetzung das 0,1-fache bis 10-fache der Gesamtmasse der schlecht wasserlöslichen Polyphenolverbindung in der Zusammensetzung beträgt.

7. Kosmetische Zusammensetzung, umfassend die Dispersionszusammensetzung gemäß einem der Ansprüche 1 bis 6.

8. Nahrungsmittelzusammensetzung, umfassend die Dispersionszusammensetzung gemäß einem der Ansprüche 1 bis 6.

9. Pharmazeutische Zusammensetzung, umfassend die Dispersionszusammensetzung gemäß einem der Ansprüche 1 bis 6.

10. Verfahren zur Herstellung der Dispersionszusammensetzung gemäß einem der Ansprüche 1 bis 6, das Verfahren umfassend:

ein Verfahren zur Herstellung einer Ölphase, bei dem eine Ölphase hergestellt wird, indem eine oder mehrere Ölphasenkomponenten, einschließlich der schlecht wasserlöslichen Polyphenolverbindung, in einem für die schlecht wasserlösliche Polyphenolverbindung guten Lösungsmittel gelöst wird oder werden; und
ein Mischverfahren, bei dem die erhaltene Ölphase und eine Phase eines für die schlecht wasserlösliche Polyphenolverbindung schlechten Lösungsmittels gemischt werden, um die Dispersionszusammensetzung zu erhalten, die die schlecht wasserlösliche Polyphenolverbindung enthält und die dispergierte Teilchen mit einer volumengemittelten Teilchengröße von 200 nm oder weniger enthält.

**11.** Verfahren zur Herstellung einer Dispersionszusammensetzung gemäß Anspruch 10, wobei das gute Lösungsmittel für die schlecht wasserlösliche Polyphenolverbindung ein wasserlösliches organisches Lösungsmittel oder eine wässrige alkalische Lösung ist.

**12.** Verfahren zur Herstellung einer Dispersionszusammensetzung gemäß Anspruch 10 oder 11, wobei das Mischen der Ölphase und der Phase des schlechten Lösungsmittels durchgeführt wird, indem die Ölphase und die Phase des schlechten Lösungsmittels jeweils durch Mikrokanäle von 1 $\mu m^2$ bis 1 $mm^2$ geleitet werden und dann die Ölphase und die Phase des schlechten Lösungsmittels vereint und vermischt werden.

**13.** Verfahren zur Herstellung einer Dispersionszusammensetzung gemäß einem der Ansprüche 10 bis 12, wobei das Mischen durch entgegengesetztes Zusammenstoßen (counter collision) erfolgt.

## Revendications

**1.** Composition de dispersion comprenant :

un composé polyphénol faiblement soluble dans l'eau ;
un émulsifiant comportant un ester d'acide gras de saccharose ; et
un polymère soluble dans l'eau,
dans laquelle,
une teneur d'un ester d'acide gras de polyglycérine dans la composition est 0, ou 0.1 ou moins de fois d'une masse totale de l'ester d'acide gras de saccharose dans la composition, et une taille de particule de particules dispersées comportant le composé polyphénol faiblement soluble dans l'eau est de 200 nm ou plus petite ;
le composé polyphénol faiblement soluble dans l'eau est au moins l'un choisi parmi le groupe constitué du resvératrol, de la curcumine, de le rutine, de l'acide ellagique et de la quercétine ; et
le polymère soluble dans l'eau est au moins l'un choisi parmi le groupe constitué des protéines et des polysaccharides.

**2.** La composition de dispersion selon la revendication 1, dans laquelle le polymère soluble dans l'eau a un poids moléculaire de 1,000 à 600,000.

**3.** La composition de dispersion selon l'une quelconque des revendications 1 ou 2, dans laquelle le polymère soluble dans l'eau est un dérivé du collagène.

**4.** La composition de dispersion selon l'une quelconque des revendications 1 à 3, dans laquelle le polymère soluble dans l'eau est au moins l'un choisi parmi le groupe constitué de la kappa-carraghénine, du dextrane et des acides hyaluroniques.

**5.** La composition de dispersion selon l'une quelconque des revendications 1 à 4, dans laquelle la teneur totale du polymère soluble dans l'eau dans la composition est de 0.1 fois à 10 fois une masse totale du composé polyphénol faiblement soluble dans l'eau dans la composition.

**6.** La composition de dispersion selon l'une quelconque des revendications 1 à 5, dans laquelle la teneur totale de l'ester d'acide gras du saccharose dans la composition est de 0.1 fois à 10 fois une masse totale du composé polyphénol faiblement soluble dans l'eau dans la composition.

**7.** Composition cosmétique comprenant la composition de dispersion selon l'une quelconque des revendications 1 à 6.

**8.** Composition alimentaire comprenant la composition de dispersion selon l'une quelconque des revendications 1 à 6.

**9.** Composition pharmaceutique comprenant la composition de dispersion selon l'une quelconque des revendications 1 à 6.

**10.** Procédé de production de la composition de dispersion selon l'une quelconque des revendications 1 6, le procédé comprenant :

un procédé de préparation d'une phase huileuse dans laquelle une phase huileuse est préparée en dissolvant un ou plusieurs composants de phase huileuse comportant le composé polyphénol faiblement soluble dans l'eau dans un bon solvant pour le composé faiblement soluble dans l'eau ; et

un procédé de mélange dans lequel la phase huileuse obtenue et une phase d'un mauvais solvant pour le composé polyphénol faiblement soluble dans l'eau sont mélangés pour obtenir la composition de dispersion qui comporte le composé polyphénol faiblement soluble dans l'eau et comporte des particules dispersées ayant une taille de particule en volume moyenne de 200 nm ou plus petite.

11. Le procédé de production d'une composition de dispersion selon la revendication 10, dans lequel le bon solvant pour le composé polyphénol faiblement soluble dans l'eau est un solvant organique soluble dans l'eau ou une solution alcaline aqueuse.

12. Le procédé de production d'une composition de dispersion selon la revendication 10 ou 11, dans lequel le mélange de la phase huileuse et de la phase du mauvais solvant est effectuée en laissant passer la phase huileuse et la phase du mauvais solvant à travers des micro-canaux de 1 $\mu$m$^2$ à 1 mm$^2$ individuellement, et ensuite en combinant et en mélangeant la phase huileuse et la phase du mauvais solvant.

13. Le procédé de production d'une composition de dispersion selon l'une quelconque des revendications 10 à 12, dans laquelle le mélange est effectué par contre-collision.

FIG.1

FIG.2

FIG.3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000136123 A
- JP 2001316259 A
- JP 2008280257 A
- JP 2005320264 A
- WO 2007060177 A1
- WO 2007060174 A1
- JP 2008154577 A
- JP 2007270073 A
- JP 2009201371 A
- JP 2005288254 A
- JP 2004033901 A

**Non-patent literature cited in the description**

- **H. NAGASAWA et al.** *Chem. Eng. Technol,* 2005, vol. 28 (3), 324-330